# EUROPEAN PATENT APPLICATION

(11) **EP 1 057 479 A1**
(43) Date of publication of application: **06.12.2000**
(21) Application number: 00115840.1
(22) Date of filing: 14.12.1995
(51) Int. Cl.: A61K 9/20, A61K 31/40, A61K 31/215, A61K 31/19, A61K 31/135

(54) **Pharmaceutical tablet formulations for direct compression**

(30) Priority: 14.12.1994 IE 940969; 14.12.1994 IE 940971; 31.07.1995 IE 950583; 31.07.1995 IE 950584; 31.07.1995 IE 950586
(62) Divisional of application: 95942265.0
(71) Applicant: ENBALT TRADING LIMITED, Dublin 2 (IE); BIOGLAN IRELAND (R & D) LIMITED, Dublin 2 (IE)
(72) Inventor: McCarthy, Paul, Clonmel, County Tipperary (IE); Donegan, Ann, Kilsheelan, County Tipperary (IE); Pattison, John, Shankill, County Dublin (IE); O'Sullivan, Richard, Kildare, County Kildare (IE)
(74) Representative: Jump, Timothy John Simon

(57) **Abstract**

A direct compression pharmaceutical tablet formulation comprising: an active pharmaceutical ingredient; a direct compression excipient which interacts with the active pharmaceutical ingredient; and an amount of an inhibitor to reduce the interaction between the direct compression excipient and the active pharmaceutical ingredient.

## Description

The invention relates to a pharmaceutical tablet formulation of an active pharmaceutical ingredient and to a method for producing such tablets.

UK-A-1,153,578 describes selegiline hydrochloride which is the compound of the formula

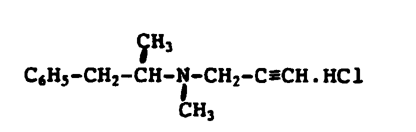

It has 5 monoamine oxidase inhibitor activity and is used in the treatment of Parkinsons disease and as a coronary dilator, hallucinogenic and anti-depressive agent and also as a tranquilliser, analgesic and an appetite suppressant.

It is known to formulate selegiline in tablet form using conventional wet granulation techniques. However, to date it has not been possible to produce a satisfactory tablet composition of selegiline by direct compression techniques. Similar problems have arisen with other active pharmaceutical ingredients.

Direct compression is the preferred technique since it is considered that fewer chemical stability problems are associated with this technique in comparison with the wet granulation process as moisture is considered to be a primary cause of instability in tablet dosage forms. In addition to the advantage of improved active ingredient stability, the use of direct compression makes it unnecessary to use applied heat to dry the damp granule. Other benefits associated with direct compression are related to particle size uniformity.

This invention therefore is directed towards a direct compression pharmaceutical tablet formulation of an active pharmaceutical ingredient.

According to the invention, there is provided a direct compression pharmaceutical tablet formulation comprising:
an active pharmaceutical ingredient,
a direct compression excipient which interacts with the active pharmaceutical ingredient, and
an amount of an inhibitor to reduce the interaction between the direct compression excipient and the active pharmaceutical ingredient.

In a preferred embodiment of the invention the formulation contains an amount of the inhibitor to substantially prevent the interaction between the direct compression excipient and the active pharmaceutical ingredient.

In one preferred embodiment of the invention the weight ratio of inhibitor to the active pharmaceutical ingredient is from 1:50 to 4:1, ideally from 8:5 to 12:5. Preferably the inhibitor includes a starch, starch-derived or cellulose-derived product, especially maize starch.

In another embodiment of the invention, the inhibitor includes a crospovidone based product.

In a preferred embodiment of the invention the excipient is direct compression lactose and/or microcrystalline cellulose.

The weight ratio of direct compression lactose to active pharmaceutical ingredient is preferably from 2:1 to 100:1 most preferably from 7:1 to 50:1, ideally from 8:1 to 11:1.

The weight ratio of microcrystalline cellulose to active pharmaceutical ingredient is preferably from 2:1 to 100:1, most preferably from 3:1 to 17:1, ideally from 4:1 to 16:1.

In one embodiment of the invention, the formulation includes a lubricant, preferably from 0.1% to 5%, most preferably approximately 0.25% by night of the formulation. The lubricant may be magnesium stearate, zinc stearate, calcium stearate, stearic acid or combinations of these materials.

In one embodiment of the invention, the formulation includes an antioxidant or other suitable stabiliser to enhance the stability of the tablet formulation. Preferably the antioxidant is present in an amount of from 0.5% to 2% by weight of the formulation.

In another embodiment of the invention the formulation may include glidants, disintegrants, fillers, wetting agents, stabilisers, binders, or combinations of these materials.

The active pharmaceutical ingredient may be selegiline or a pharmaceutically acceptable salt thereof, preferably selegiline hydrochloride. The active pharmaceutical ingredient may also be other compounds including oxybutynin or a pharmaceutically acceptable salt thereof, preferably oxybutynin chloride; bumetanide or a pharmaceutically acceptable salt thereof; or indapamide or a pharmaceutically acceptable salt thereof, preferably indapamide hemihydrate.

The invention also provides a method of preparing a tablet by preparing a formulation according to the invention and directly compressing said formulation to form a tablet.

The invention further provides a method for producing tablets of an active pharmaceutical ingredient comprising the steps of:-
mixing the active pharmaceutical ingredient, a lubricant, a direct compression excipient, and an mount of an inhibitor to substantially prevent interaction between the direct compression lactose and the active pharmaceutical ingredient; and
directly compressing the mixture thus formed to form tablets.

Preferably the formulation includes a lubricant and the method includes the step of mixing the ingredients except the lubricant and subsequently adding the lubricant. Preferably the mixing is a dry blending technique. The excipient is preferably direct compression lactose.

The inhibitor preferably includes a starch, starch-derived or cellulose-derived product. Alternatively, the inhibitor includes a crospovidone based product.

The invention will be more clearly understood from the following description thereof given by way of example only.

In each case, the formulation is a direct compression formulation. The ingredients were weighed out and all the ingredients except the lubricant were added to a receptacle and dry blended for about 6 minutes. The lubricant (in these cases magnesium stearate) was then added and the mixture is blended for a further 2 minutes. Tablets were then formed from the blend by conventional direct compression techniques.

In Examples A and 1 to 4, selegiline hydrochloride was used as the active ingredient. However, it will be appreciated that selegiline base or any pharmaceutically acceptable salt thereof may be used as the active ingredient. Similar comments apply to the examples referring to other active ingredients. In examples B and 5 oxybutynin chloride was used. In example 9 indapamide hemihydrate was used.

All of the excipients used in the examples are of pharmaceutical grade.
- Magnesium Stearate:: consists mainly of magnesium stearate with variable proportions of magnesium palmitate and/or magnesium oleate. It is a tablet lubricant.
- Zinc Stearate:: consists mainly of zinc stearate with variable proportions of zinc palmitate and zinc oleate. It is a tablet lubricant.
- Sodium Lauryl Sulfate:: an anionic surfactant used as a detergent and wetting agent in tablet formulations.
- Croscarmellose Sodium:: is a cross-linked polymer of carboxymethylcellulose sodium. It is a tablet disintegrant.
- Sodium Starch Glycolate:: is the sodium salt of a poly-α-glucopyranose in which some of the hydroxyl groups are in the form of carboxymethyl ether. It is a tablet disintegrant.
- Crospovidone:: is a water insoluble synthetic cross-linked homopolymer of N-vinyl-2-pyrollidone. It is a tablet disintegrant.
- Microcrystalline Cellulose:: is a purified, partially depolymerised cellulose and is used as a diluent and has some lubricant and disintegrant properties.
- Direct Compression Lactose (DC Lactose):: is direct compression grade of lactose which is more fluid and more compressible than crystalline or powdered lactose.

Various proprietary brands of direct compression lactose are commercially available. In the examples given below, the brand of direct compression lactose used was Tablettose 80 available from Meggle GmbH of Germany. We have also achieved similar results using Pharmatose DC11 which is available from DMV International of The Netherlands.

It will be appreciated that various similar formulations to those described below may be prepared by scaling up or down the components of the mixture so that the new formulation is quantitatively proportional to those given below. For example, formulations containing 10 mgs of selegiline may be prepared in this way. Similarly, an oxybutynin formulation may be prepared in this way with 2.5 mg rather than 5 mg of active. In addition, a bumetanide formulation may also be prepared in this way with 1 mg rather than 5 mg of active ingredient.

### EXAMPLE A

A batch of tablets were prepared by direct compression. Each tablet had the following composition:-
- 5 mg: selegiline hydrochloride
- 85 mg: DC Lactose

The tablets gave an assay of 91.8%.

This example illustrates the low assay that results when tablets of selegiline are manufactured by direct compression techniques using direct compression lactose.

### EXAMPLE 1

A batch of tablets were prepared by direct compression. Each tablet had the following composition:-
- 5 mg: selegiline hydrochloride
- 52.75 mg: DC Lactose
- 24 mg: microcrystalline cellulose
- 8 mg: maize starch
- 0.25 mg: magnesium stearate

The tablets gave an assay of 99.5%.

It will be noted that the use of an inhibitor, in this case maize starch, radically improves the assay result.

### EXAMPLE 2

A batch of tablets were prepared by direct compression. Each tablet had the following composition:-
- 5 mg: selegiline hydrochloride
- 48.75 mg: DC Lactose
- 24 mg: microcrystalline cellulose
- 12 mg: maize starch
- 0.25 mg: magnesium stearate

The tablets gave an assay of 97.94%.

Using a larger mount of maize starch and less DC Lactose did not significantly effect the assay.

### EXAMPLE 3

A batch of tablets were prepared by direct compression. Each tablet had the following composition:-
- 5 mg: selegiline hydrochloride
- 48.75 mg: DC Lactose
- 24 mg: microcrystalline cellulose
- 11 mg: maize starch
- 1 mg: citric acid
- 0.25 mg: magnesium stearate

The tablets gave an assay of 99.6%.

The addition of an antioxidant, in this case citric acid, is to counteract any oxidation reactions in storage.

### EXAMPLE 4

A batch of tablets were prepared by direct compression. Each tablet had the following composition:-
- 5 mg: selegiline hydrochloride
- 49.45 mg: Direct Compression Lactose
- 24 mg: Microcrystalline Cellulose
- 11 mg: Maize Starch
- 0.55 mg: Zinc Stearate

The tablets gave an assay of 100.7%

From extensive research and development in producing tablets of selegiline by direct compression techniques, we have noticed a very significant interaction between selegiline hydrochloride and conventional bulking agents such as DC Lactose and microcrystalline cellulose. It has not been possible using conventional formulations to produce a tablet of selegiline by direct compression without a very significant adverse affect on the assay results for the tablets produced. In all cases, the assay was found to be less than 95%, in some cases, as low as 70%. It is not fully understood why such poor assay results were obtained. It is, however, speculated that there may be an interaction between the selegiline and the DC Lactose and microcrystalline cellulose, resulting possibly in the formation of a complex which adversely affects the amount of selegiline detectable or available on assay. We have surprisingly found that the interaction is avoided and good assay results are obtained if an inhibitor, particularly maize starch, is incorporated especially in the amounts mentioned above.

It will be appreciated that it may be possible to use direct compression excipients either in addition to or as an alternative either to direct compression lactose or microcrystalline cellulose. Such direct compression excipients include calcium hydrogen phosphate, compressible sugar, dextrates, and pregelatinized starch.

It will be appreciated that the formulation may include any suitable lubricant such as magnesium stearate or zinc stearate as described above or other stearic acid salts such as calcium stearate. Other suitable lubricants may also be incorporated, including glyceryl monostearate, glyceryl palmitostearate, hydrogenated castor oil, hydrogenated vegetable oil type 1, light mineral oil, mineral oil, polyethylene glycol, sodium benzoate, sodium lauryl sulphate, sodium stearyl fumarate, stearic acid and talc.

It will also be appreciated that while the presence of an antioxidant may not be essential, in cases where such an antioxidant is used it may be selected from one or more of the following: alpha tocopherol, ascorbic acid, ascorbyl palmitate, butylated hydroxyanisole, butylated hydroxytoluene, citric acid, fumaric acid, malic acid, propylgallate, sodium ascorbate, sodium metabisulphite.

We have also found that the interactions described above also apply to other active pharmaceutical ingredients. The following examples illustrate how similar techniques may also be applied to direct compression pharmaceutical tablet formulations of active ingredients other than selegiline.

### EXAMPLE B - COMPARISON

A batch of tablets were prepared by direct compression. Each tablet had the following composition:-
- 5 mg: oxybutynin chloride
- 174 mg: DC Lactose
- 1 mg: Magnesium Stearate

The tablets gave an assay of 88.3%.

### EXAMPLE 5

A batch of tablets were prepared by direct compression. Each tablet had the following composition:-
- 5 mg: oxybutynin chloride
- 119 mg: DC Lactose
- 43 mg: microcrystalline cellulose
- 12 mg: crospovidone
- 1 mg: magnesium stearate

The tablets gave an assay of 97.9%.

### EXAMPLE C - COMPARISON

A batch of tablets were prepared by direct compression. Each tablet had the following composition.
- 5 mg: bumetanide
- 343 mg: DC Lactose
- 2 mg: magnesium Stearate

The tablets gave an assay of 91%.

### EXAMPLE D - COMPARISON

A batch of tablets were prepared by direct compression. Each tablet had the following composition.
- 5 mg: bumetanide
- 343 mg: microcrystaline cellulose
- 2 mg: magnesium stearate

The tablets gave a assay of 93.7%.

### EXAMPLE 6

A batch of tablets were prepared by direct compression. Each tablet had the following composition.
- 5 mg: bumetanide
- 237.5 mg: DC Lactose
- 80 mg: microcrystalline cellulose
- 25 mg: crospovidone
- 2.5 mg: magnesium stearate

The tablets gave an assay of 97.4%.

### EXAMPLE 7

A batch of tablets were prepared by direct compression. Each tablet had the following composition.
- 5 mg: bumetanide
- 237.5 mg: DC Lactose
- 80 mg: microcrystalline cellulose
- 25 mg: maize starch
- 2.5 mg: magnesium stearate

The tablets gave an assay of 98.2%.

### EXAMPLE 8

A batch of tablets were prepared by direct compression. Each tablet had the following composition.
- 5 mg: bumetanide
- 241 mg: DC Lactose
- 80 mg: microcrystalline cellulose
- 17 mg: croscarmellose sodium
- 5 mg: sodium lauryl sulphate
- 2 mg: magnesium stearate

The tablets gave an assay of 97.9%

### EXAMPLE 9

A batch of tablets were prepared by direct compression. Each tablet had the following composition:-
- 2.5 mg: indapamide hemihydrate
- 82.75 mg: DC Lactose
- 4 mg: carboxymethyl starch
- 0.75 mg: magnesium stearate.

The tablets gave an assay of 100.4%.

It will be appreciated that similar techniques may be applied to produce direct compression tablet formulations of other active pharmaceutical ingredients which interact with direct compression excipients.

The invention is not limited to the embodiments hereinbefore described which may be varied in detail.

## Claims

1. A direct compression pharmaceutical tablet formulation comprising:
an active pharmaceutical ingredient;
a direct compression excipient which interacts with the active pharmaceutical ingredient; and
an amount of an inhibitor to reduce the interaction between the direct compression excipient and the active pharmaceutical ingredient.

2. A direct compression pharmaceutical tablet formulation as claimed in claim 1 wherein the formulation contains an amount of inhibitor to substantially prevent the interaction between the direct compression excipient and the active pharmaceutical ingredient.

3. A formulation as claimed in claim 1 or claim 2 wherein the weight ratio of inhibitor to the active pharmaceutical ingredient is from 1:50 to 4:1 and, preferably, from 8:5 to 12:5.

4. A formulation as claimed in any preceding claim wherein the inhibitor includes a starch, a starch-derived or cellulose-derived product, preferably maize starch, and/or a crospovidone based product.

5. A formulation as claimed in any preceding claim wherein the excipient includes direct compression lactose, and/or microcrystalline cellulose.

6. A formulation as claimed in claim 5 wherein the weight ratio of direct compression lactose to the active pharmaceutical ingredient is from 2:1 to 100:1, from 7:1 to 50:1, or from 8:1 to 11:1.

7. A formulation as claimed in claim 5 wherein the weight ratio of microcrystalline cellulose to active pharmaceutical ingredient is from 2:1 to 100:1, from 3:1 to 17:1, or from 4:1 to 16:1.

8. A formulation as claimed in any preceding claims including a lubricant, optionally in an amount from 0.1% to 5% by weight of the formulation, and preferably in an amount of approximately 0.25% by weight of the formulation.

9. A formulation as claimed in any preceding claim including an antioxidant or other suitable stabiliser to enhance the stability of the tablet formulation, the antioxidant preferably being present in an amount from 0.5% to 2% by weight of the formulation.

10. A formulation as claimed in any preceding claim including either a glidant, a disintegrant, a filler, a wetting agent, stabiliser, a binder or any combination of these materials.

11. A formulation as claimed in any preceding claim wherein the active pharmaceutical ingredient is:
(a) selegiline or a pharmaceutically acceptable salt thereof, preferably selegiline hydrochloride;
(b) oxybutynin or a pharmaceutically acceptable salt thereof, preferably oxybutynin chloride;
(c) bumetanide or a pharmaceutically acceptable salt thereof; or
(d) indapamide or a pharmaceutically acceptable salt thereof, preferablt indapamide hemihydrate.

12. A method of preparing a tablet comprising;
preparing a formulation as claimed in any preceding claim; and
directly compressing said formulation into a tablet.

13. A method for producing tablets of an active pharmaceutical ingredient comprising the steps of:-
mixing the active pharmaceutical ingredient, a lubricant, a direct compression excipient, and an amount of an inhibitor to reduce interaction between the direct compression excipient and the active pharmaceutical excipient; and
directly compressing the mixture thus formed to form tablets.

14. A method as claimed in claim 13 wherein the method includes the steps of:
mixing the ingredients except the lubricant;
subsequently adding the lubricant; and
further mixing the ingredients.

15. A method as claimed in claim 13 or 14 wherein the mixing is performed using a dry blending technique.

16. A method as claimed in any of claims 13 to 15 wherein the direct compression excipient includes direct compression lactose.

17. A method as claimed in any of claims 13 to 16 wherein the inhibitor includes a starch, a starch-derived product, a cellulose-derived product, a derivative thereof, or a crosprovidone based product.

18. Tablets of an active pharmaceutical ingredient whenever produced by a method as claimed in any of claims 12 to 17.

19. Tablets of:
(a) selegiline or a pharmaceutically acceptable salt thereof, preferably selegiline hydrochloride;
(b) oxybutynin or a pharmaceutically acceptable salt thereof, preferably oxybutynin chloride;
(c) bumetanide or a pharmaceutically acceptable salt thereof; or
(d) indapamide or a pharmaceutically acceptable salt thereof, preferablt indapamide hemihydrate
whenever produced by a method as claimed in any of claims 12-17.

20. A tablet prepared or preparable from a formulation as claimed in any of claims 1 to 11.
